# EUROPÄISCHE PATENTSCHRIFT

(11) **EP 0 571 940 B1**
(45) Veröffentlichungstag und Bekanntmachung des Hinweises auf die Patenterteilung: **05.11.1997**
(21) Anmeldenummer: 93108404.0
(22) Anmeldetag: 25.05.1993
(51) Int. Cl.: G01N 33/52, B29C 43/36, B29C 43/46

(54) **Testträger zur Analytbestimmung sowie Verfahren zu dessen Herstellung**
Test strip for analyte detection and method for manufacturing the same
Support d'essai pour la détection d'analytes et procédé de fabrication dudit support

(30) Priorität: 29.05.1992 DE 4217733
(43) Veröffentlichungstag der Anmeldung: 01.12.1993
(73) Patentinhaber: BOEHRINGER MANNHEIM GMBH, 68298 Mannheim (DE)
(72) Erfinder: Macho, Heinz, W-6149 Fürth-Fahrenbach (DE); Lerch, Rolf, W-6804 Ilvesheim (DE); Harttig, Herbert, Dr., W-6701 Altrip (DE); Zimmer, Volker, W-6700 Ludwigshafen-Edigheim (DE)

(56) Entgegenhaltungen:
- EP-A- 0 126 357
- EP-A- 0 185 982
- EP-A- 0 199 205
- EP-A- 0 209 032
- EP-A- 0 245 767
- EP-A- 0 443 231
- DE-A- 3 442 820

## Beschreibung

Die Erfindung betrifft Testträger zur Analytbestimmung mit thermisch erzeugten Begrenzungen zur Lenkung eines Flüssigkeitsstromes in einem saugfähigen Material von einem Probenaufgabefeld zu einem Nachweisfeld, wobei durch die Begrenzungen hindurch kein Probenflüssigkeitstransport stattfinden kann. Außerdem betrifft die Erfindung ein Verfahren zur Herstellung eines solchen Testträgers.

Unter Testträgern werden Analysenelemente verstanden, bei denen sich die zur Durchführung der Bestimmung eines Probenbestandteils notwendigen Reagenzien in oder auf festen Materialien befinden. Solche Trägermaterialien können im allgemeinen saugfähige faserige, poröse oder quellbare Materialien sein, wie Vliese, Gewebe, Membranen oder filmartige Materialien. Papier ist beispielsweise ein gebräuchliches saugfähiges Trägermaterial. Die Trägermaterialien selbst können steif und damit selbsttragend sein oder sie können auch auf einer steifen, beispielsweise auf einer Plastikunterlage befestigt sein. Da sich in Testträgern die zur Bestimmung eines Analyts notwendigen Reagenzien beispielsweise in beschichteter oder imprägnierter Form auf oder in trockenen Reagenzträgermaterialien befinden, werden Bestimmungen in Anspielung auf die Einsatzform der Reagenzien oft auch als trockenchemische Tests bezeichnet. Testträger sind dem Fachmann in vielerlei Formen, beispielsweise als Streifen, quadratische Plättchen etc. bekannt.

Deutsch et al. beschreiben in US-Patent 4,235,601 Testträger, die als einschichtige Eintauchteststreifen vorliegen. Auf Papier sind an bestimmten Stellen Reagenzien imprägniert. Wird ein solcher Papierstreifen nach Probenaufgabe in Chromatographieflüssigkeit gestellt, gelangt mit der wandernden Flüssigkeit die Probe nacheinander mit den auf dem Papier befindlichen Reagenzien in Berührung. Die unterschiedlichen Chromatographieeigenschaften des Produkts aus nachzuweisendem Analyt und Reagenz sowie überschüssigem Reagenz und nichtinteressierenden Beiprodukten und Probenbestandteilen ermöglichen letztendlich eine Bestimmung des Analyts in der zu untersuchenden Probe an einer bestimmten Stelle des Teststreifens. Um die oft nur geringfügig unterschiedlichen Chromatographieeigenschaften zum Tragen zu bringen, sind relativ lange Chromatographiewege erforderlich. Damit verbunden, ist ein relativ hoher Flüssigkeitsbedarf notwendig, um ein Durchwandern aller Zonen des Teststreifens zu ermöglichen.

Weniger Flüssigkeit ist im allgemeinen bei mehrschichtigen Testträgern erforderlich, bei denen die einzelnen Schichten übereinander angeordnet sind und verschiedene Aufgaben erfüllen. So können solche Testträger beispielsweise eine Verteilungsschicht, Reagenzschicht(en), evtl. eine optische Sperrschicht, eine Trägerschicht etc. beinhalten. Solche Testträger sind beispielsweise aus DE-A-3222366 oder EP-A-0 166365 bekannt.

Eine flüssige Probe wird auf die oberste Schicht aufgegeben und difundiert dabei sowohl in die Breite als auch in die Tiefe. Vorteilhafterweise wird das gesamte Schichtenmaterial befeuchtet. Durch Reaktion des Analytes mit den in einer oder mehreren Schichten vorliegenden Reagenzien wird in der Regel eine Farbe gebildet oder verändert. Diese Farbbildung oder -änderung als Indiz für die Anwesenheit des zu bestimmenden Analyts kann visuell beobachtet oder photometrisch quantitativ gemessen werden und so für die quantitative Bestimmung eines Analyten genutzt werden.

In DE-A-2934760 werden mehrschichtige Reagenzträger beschrieben, die für die gleichzeitige Durchführung verschiedener Analysen geeignet sein sollen. Hierfür wird ein sogenanntes "integriertes Material" vorgeschlagen, das drei oder mehr "Reagenzschichteinheiten" auf einem Träger sowie eine poröse Ausbreitungs- bzw. Verteilungsschicht aufweist. Die jeweiligen Reagenzschichteinheiten enthalten Reagenzien für verschiedene chemische Analysen. Sie sind auf einer Trägerschicht so angeordnet, daß annähernd gleiche Anteile davon sich innerhalb eines Verteilungskreises befinden, der durch die Diffussion einer flüssigen Probe, wenn sie durch die poröse Verteilungsschicht hindurch gelangt, definiert ist. Das gesamte Schichtenmaterial, außer der Trägerschicht, muß für die zu untersuchende Flüssigkeit durch Diffussion zugänglich sein. Unter "Diffussion" wird ganz allgemein das Hineinwandern von Flüssigkeit in das Schichtenmaterial verstanden, sei es durch Kapillarkräfte, Schwerkraft etc. Gegebenenfalls können radial angeordnete Reagenzschichteinheiten so angeordnet sein, daß sie sich nicht gegenseitig berühren und ein freier Raum zwischen ihnen verbleibt. Dieser freie Raum kann so behandelt werden, daß er wasserfest oder wasserabstoßend ist. Auf eine so behandelte zentrale Stelle aufgegebene wässrige Flüssigkeit wird dann von den angrenzenden Reagenzschichteinheiten aufgesaugt.

Aus EP-B-0 209 032 sind mehrschichtige als Analysenelemente bzeichnete Testträger bekannt, bei denen vermieden werden soll, daß Flüssigkeit aus einer Testmatrix in eine andere Testmatrix auf dem selben Testträger gelangen soll. Hierzu besteht das mehrschichtige Analysenelement mindestens aus einer Reagenz enthaltenden Schicht und einer benachbarten in der Wärme schmelzbaren Schicht. Beim Schneiden eines solchen Schichtenaufbaus mit Ultraschall und/oder Laserenergie werden die Schichten an ihren Kanten zusammengeschweißt. So werden die Kanten quasi versiegelt und ein überlaufen von Flüssigkeit von einer Testmatrix zu einer anderen auf dem gleichen Testträger soll so vermieden werden.

In EP-A-0 185 982 werden insbesondere Eintauchteststreifen beschrieben, die für den gleichzeitigen Nachweis mehrerer Parameter geeignet sein sollen. Hierzu müssen auf einem Testträger nebeneinander in Testfeldern die verschiedenen, für den Nachweis der jeweiligen Parameter notwendigen Reagenzien vorliegen. Das Überlaufen von Flüssigkeit aus einem Testfeld in die benachbarten kann zu Störungen führen. Um dieses Überlaufen zu unterbinden, wird vorgeschlagen offenzellige, natürliche oder synthetische Materialien als Reagenzträgermaterialien zu verwenden, bei denen durch Wärme- oder Ultraschallbehandlungen an bestimmten Stellen die Zellöffnungen geschlossen werden können, so daß separate Reagenzmatrices erhalten werden, die effektiv voneinander isoliert sind.

Während es aus dem vorstehenden Stand der Technik bekannt war, durch lokale Wärmebehandlung von Testmatrices, d. h. Trägermaterialien, Begrenzungen in mehrschichtigen Testvorrichtungen zu erzeugen, durch die ein Flüssigkeitsfluß von einem Ort an einen anderen verhindert werden soll, betrifft EP-A-0 443 231 mehrschichtige sogenannte "Testkarten" mit Begrenzungen für den Fluß von Flüssigkeiten, um die Probenflüssigkeit von einem Ort zu einem anderen zu lenken. Solche Flüssigkeitsbegrenzungen können unter anderem Begrenzungen sein, die durch Ultraschall oder Wärme erzeugt worden sind oder Begrenzungen, die durch Verschweißen entstanden sind. Als wesentliche Merkmale der mehrschichtigen Testkarten wird die Sog- und Pumpwirkung zwischen zwei verschiedenporigen Schichten betont. Eine erste Schicht muß Flüssigkeit gut in eine zweite zur Kooperation notwendige Schicht leiten können. Hierzu ist es erforderlich, daß diese zweite Schicht Flüssigkeit gut aufzusaugen vermag. Innerhalb der einzelnen Schichten sind die verschiedenen Funktionszonen durch Flüssigkeitsbegrenzungen vollständig voneinander isoliert. Nur durch Übereinanderlegen von Schichten in der Weise, daß überlappende, Flüssigkeit leitende Bezirke zwischen den Schichten erzeugt werden, ist es möglich, daß Flüssigkeit von einer ersten Schicht in eine zweite Schicht und von dort zurück in einer vom Startpunkt verschiedene Zone der ersten Schicht gelangt. Somit befinden sich die verschiedenen Funktionszonen in verschiedenen Schichten oder sind innerhalb der gleichen Schicht durch Flüssigkeitsbegrenzungen vollständig voneinander getrennt. Soweit ersichtlich, stehen so innerhalb der mehrschichtigen Testkarte mit Reagenz imprägnierte Zonen verschiedener Schichten in direktem Kontakt miteinander.

Allen mehrschichtigen Testträgern bzw. Analysenelementen zur Bestimmung eines Analyts gemeinsam ist ihr aus produktionstechnischer Sicht komplizierter Aufbau, da in der Regel jede Schicht separat behandelt und zurechtgeschnitten werden muß, bevor die Schichten letztendlich zu einem Testträger zusammengefügt werden.

Die der vorliegenden Erfindung zugrunde liegende Aufgabe ist deshalb in der Bereitstellung möglichst einfach zu fertigender, mit kleinem Probevolumen zuverlässig arbeitenden, für den Benutzer gut handhabbaren und auswertbaren Testträgern zu sehen.

Diese Aufgabe wird durch die Erfindung, wie sie in den Patentansprüchen gekennzeichnet ist, gelöst.

Gegenstand der Erfindung ist ein Testträger zur Bestimmung eines Analyts in einer flüssigen Probe enthaltend eine Schicht eines flächig ausgebildeten Materials mit mindestens einem saugfähigen Probenaufgabenbereich und mindestens einem saugfähigen Nachweisbereich mit für die Bestimmung des Analyts erforderlichem Reagenz sowie gegebenenfalls noch einem oder mehreren weiteren saugfähigen Funktionsbereichen, wobei zur Lenkung von zu untersuchender Probeflüssigkeit vom Probenaufgabenbereich zum Nachweisbereich gegebenenfalls über ein oder mehrere weitere saugfähige Funktionsbereiche innerhalb des flächig ausgebildeten Materials Begrenzungen vorliegen, durch die hindurch kein Probenflüssigkeitstransport stattfinden kann. Der erfindungsgemäße Testträger ist dadurch gekennzeichnet, daß sich alle Funktionsbereiche zur Bestimmung eines Analyts innerhalb einer einzigen Schicht des flächig ausgebildeten Materials befinden und die einzelnen Funktionsbereiche sich nicht direkt berühren, sondern durch saugfähige Stege des flächig ausgebildeten Materials getrennt sind. Erfindungsgemäß sind Probenaufgabe-, Reagenz enthaltender Nachweisbereich sowie gegebenenfalls weitere saugfähige Funktionsbereiche und saugfähige Stege durch eine insgesamt in der Fläche allseitige Begrenzung definiert durch die diese Bereiche und die Stege so vom Rest des flächig ausgebildeten Materials abgetrennt sind, daß zwischen Probenaufgabe- und Nachweisbereich über den oder die saugfäigen Stege sowie gegebenenfalls weitere saugfähige Funktionsbereiche ein Flüssigkeitstransport erfolgen kann, Flüssigkeitstransport aus einem dieser Bereiche oder dem oder den Stegen in das außerhalb der Begrenzung liegende restliche flächig ausgebildete Material jedoch verhindert ist.

Weiter ist Gegenstand der Erfindung ein Verfahren zur Herstellung eines erfindungsgemäßen Testträgers, das dadurch gekennzeichnet ist, daß in einer Schicht eines saugfähigen Materials durch Wärmebehandlung das um die saugfähigen Bereiche und die sie verbindenden Stege herum befindliche Material so verändert wird, daß es nicht mehr saugfähig ist.

Der wesentliche Teil eines erfindungsgemäßen Testträgers ist in der Schicht des flächig ausgebildeten Materials zu sehen, in der sich die Funktionsbereiche und die sie verbindenden Stege befinden. Als Funktionsbereiche sollen mindestens vorhanden sein: ein Probenaufgabebereich, das heißt, eine Zone saugfähigen Materials auf das die zu bestimmende Probe aufgegeben werden soll und mindestens ein Nachweisbereich, der entweder das gesamte oder einen Teil des zur Bestimmung des Analyts notwendigen Reagenzes enthält. Gegebenenfalls enthält die Schicht auch ein oder mehrere Vorreaktionsbereiche, das heißt, Zonen, die entweder reagenzfrei oder einen Teil des zur Bestimmung notwendigen Reagenzes enthalten, der nicht im Nachweisbereich vorliegt. Auch der Probenaufgabenbereich kann einen Teil des zum Nachweis des zu bestimmenden Analyts notwendigen Reagenzes enthalten. Insofern kann ein reagenzfreier Vorreaktionsbereich zur Inkubation von Flüssigkeit dienen, die im Probenaufgabenbereich mit Reagenz kontaktiert und gemischt wurde.

Die Funktionsbereiche innerhalb der Trägermaterialschicht sollen sich nicht direkt berühren, sondern sollen durch saugfähige Stege des flächig ausgebildeten Materials getrennt sein.

Saugfähige Funktionsbereiche und sie verbindenden saugfähigen Stege sind erfindungsgemäß innerhalb des flächig ausgebildeten Trägermaterials durch eine Begrenzung von dem umgebenden Schichtenmaterial insofern isoliert, als durch die Begrenzung hindurch kein Probenflüssigkeitstransport stattfinden kann. Probenflüssigkeit kann sich deshalb nur innerhalb des abgegrenzten Bereiches, das heißt, in den Funktionsbereichen und auf den dazwischen befindlichen Stegen bewegen.

Als flächig ausgebildetes Trägermaterial für erfindungsgemäße Testträger kommen grundsätzlich alle saugfähigen Materialien, insbesondere faserige oder poröse Materialien in Frage, die die zu untersuchende Probenflüssigkeiten aufsaugen können und in denen sich diese Flüssigkeit, analog wie aus der Chromatographie bekannt, bewegen kann. In faserigen Materialien können die einzelnen Fasern ungeordnet vorliegen, wie beispielsweise in Vliesen oder die Fasern können geordnet vorliegen, wie es beispielsweise in Geweben der Fall ist. Als poröse Materialien können poröse Filme oder Membranen eingesetzt werden. Erfindungsgemäß besonders bevorzugt sind solche saugfähigen Materialien, in denen die Flüssigkeitsbegrenzung um die Funktionsbereiche und die sie verbindenden Stege herum ohne Zuhilfenahme eines weiteren Materials durch Wärme erzeugt werden kann. Dies ist beispielsweise möglich, wenn das gesamte saugfähige Material an sich in der Wärme verformbar ist, oder wenn das saugfähige Material in der Wärme verformbare Bestandteile enthält, die zur Ausbildung einer flüssigkeitsundurchlässigen Begrenzung im saugfähigen Material ausreicht. Insofern sind saugfähige Schichten, die entweder ganz oder teilweise aus thermoplastischen Kunststoffen, wie Polyamid, Polyester, Celluloseester, Polypropylen oder ähnlichem aufgebaut sind besonders bevorzugt. Ganz besonders bewährt hat sich für den Einsatz in einem erfindungsgemäßen Testträger ein Vlies aus 30 Teilen Polyester, 20 Teilen Zellwolle, 30 Teilen Copolyester und 20 Teilen Polyvinylalkohol.

Die vorstehend beschriebenen Trägermaterialien können durch lokale Wärmebehandlung so verändert werden, daß sie an diesen Stellen nicht mehr saugfähig und für Flüssigkeit praktisch undurchlässig sind. Für eine solche Wärmebehandlung ist Laserenergie, Ultraschall oder jede andere Art von Wärme erzeugenden oder warmen Mitteln geeignet. Als besonders bevorzugt erwiesen haben sich Prägewerkzeuge, wie beispielsweise Stempel oder Walzen, die selbst warm sind oder mit Hilfe von Wärme erzeugenden Mitteln oder Energien, wie beispielsweise Ultraschall betrieben werden. Solche Stempel oder Walzen besitzen Erhebungen und Vertiefungen, wobei die Erhebungen beim Aufdrücken des Stempels oder der Walze mit der Prägeseite auf die saugfähige Schicht, die für Flüssigkeit nicht durchlässige Begrenzung um die Funktionsbereiche und die sie verbindenden Stege herum erzeugen. Beim Aufdrücken des Stempels oder der Walze auf die saugfähige Schicht wird nämlich das Schichtenmaterial an den warmen Stellen der Stempelerhebungen geschmolzen oder zumindest gesintert, so daß innerhalb dieser Stellen kein Flüssigkeitstransport mehr möglich ist. Die Vertiefungen auf der Prägeseite des Stempels entsprechen den Funktionsbereichen und den sie verbindenden Stegen. Die Vertiefungen müssen deshalb so tief sein, daß sie beim Aufdrücken der Prägeseite des Stempels auf das saugfähige Material die Saugfähigkeit nicht wesentlich beeinträchtigen. Als Stempel- bzw. Walzenmaterialien sind aus dem Werkzeugbau bekannte Metalle und Legierungen mit ausreichend guter Wärmeleitfähigkeit geeignet. Vorzugsweise sollte die Stempel- bzw. Walzenfläche mit der die Prägung erfolgt, antiadhäsiv sein. Dies kann beispielsweise durch Teflonisieren erfolgen.

Erfindungsgemäß ist es auch möglich, die flüssigkeitsundurchlässige Begrenzung um die Funktionsbereiche und die sie verbindenen Stege herum mit einem von der saugfähigen Schicht verschiedenen Material zu erzeugen. Beispielsweise ist es möglich, eine saugfähige Schicht aus nicht in der Wärme verformbarem Material mit einem in der Wärme verformbarem Material zu unterlegen. Bei lokaler Wärmebehandlung des durch Wärme verformbaren Materials durch die saugfähige durch Wärme nicht verformbare Schicht hindurch ist es möglich, daß gegebenenfalls auch unter Druck das in der Wärme verformbare Material in die saugfähige Schicht eindringt und diese an den dortigen Stellen für Flüssigkeit undurchlässig macht. So ist es beispielsweise möglich, eine saugfähige Schicht aus nicht in der Wärme verformbaren Material, mit einer Schicht aus in der Wärme verformbarem Material zu unterlegen und mit einem wie vorstehend beschriebenen warmen oder mit Wärme erzeugenden Mitteln betriebenem Prägewerkzeug so auf die saugfähige Schicht zu drücken, daß an den Stempel- bzw. Walzenerhebungen das darunterliegende in der Wärme verformbare Material so verändert wird, daß es in die saugfähige Schicht gelangt und diese dort für Flüssigkeit undurchlässig macht.

Letztgenannte Methode kann insbesondere auch dann angewendet werden, wenn das saugfähige Schichtenmaterial allein nur eine geringe Eigensteifigkeit aufweist und so zur Stabilisierung und besseren Handhabung dieses Materials mit einer Tragschicht kombiniert werden soll.

Auf diese Art und Weise können beispielsweise Zellstoffvliese, Gewebe aus monofilen oder multifilen, schwer schmelzbaren Garnen, Glasfasergewebe oder Glasfaservliese mit einer mit Schmelzkleber beschichteten, selbst temperaturbeständigen Folie kombiniert werden. Als Schmelzkleber eignen sich insbesondere die Elvax-Typen der Firma DuPont oder die Dynapol-Typen der Fa. Dynamit Nobel. Als temperatur- und formstabile Trägerfolien lassen sich Polyesterfolien gut verwenden.

Die die Flüssigkeit lenkende Begrenzung innerhalb der saugfähigen Schicht kann dünn sein und lediglich so etwas wie eine Umrandung der Funktionsbereiche und der sie verbindenden Stege darstellen. Sie kann aber auch so ausgestaltet sein, daß innerhalb der gesamten Schicht nur noch die Funktionsbereiche und die sie verbindenden Stege die saugfähige Eigenschaft des Materials besitzen. Wesentlich ist in jedem Fall vor allem, daß Flüssigkeit nicht außerhalb der Funktionsbereiche und der sie verbindenden Stege gelangt.

Die Funktionsbereiche können die verschiedensten Formen aufweisen. Sie können rund, oval, rechteckig, quadratisch sein oder sonstige Formen besitzen, sofern dies für die Funktion des Feldes zweckmäßig erscheint. Als besonders geeignet haben sich runde Funktionsbereiche erwiesen. Die Größe der Funktionsbereiche kann ebenfalls den Bedürfnissen in weiten Bereichen angepaßt werden. Zu beachten ist, daß große flächige Ausdehnungen der Funktionsbereiche größere Proben- oder Flüssigkeitsvolumina erfordern als kleinere Bereichsausdehnungen. Für die erfindungsgemäßen Testträger bevorzugt, haben sich Flächen von 10 mm² bis 50 mm² der Funktionsbereiche erwiesen.

Die die Funktionsbereiche verbindenden saugfähigen Stege sind verglichen mit der Breite oder dem Durchmesser der durch sie verbundenen Funktionsbereiche in der flächigen Ausdehnung dünne Bezirke des saugfähigen Schichtenmaterials. Die Stege sind in der Regel reagenzfrei. Vorzugsweise besitzen sie eine Breite von 0,5 mm bis 3,0 mm und eine Länge von 2 mm bis 10 mm.

Die Dicke der saugfähigen Schicht innerhalb der Funktionsbereiche und der sie verbindenden Stege entspricht in etwa der Dicke des ursprünglich für die Herstellung des erfindungsgemäßen Testträger eingesetzten saugfähigen Schichtenmaterials und beträgt etwa 0,1 mm bis 1,0 mm. Auch hier gilt, daß Abweichungen nach oben oder unten möglich sind, je nach dem, ob mit mehr oder weniger Flüssigkeit gearbeitet werden kann oder soll. Im übrigen ist durch die Wahl der Dicke des saugfähigen Schichtenmaterials und der Form und den Abmessungen der Funktionsbereiche und den sie verbindenden Stegen eine Steuerung des zeitlichen Verlaufs des Bestimmungsverfahrens auf dem Testträger ohne äußere Eingriffe möglich. So benötigt ein bestimmtes Flüssigkeitsvolumen mehr Zeit einen großen Funktionsbereich zu füllen als es für einen kleinen Funktionsbereich notwendig ist. Lange Stege führen zu einer größeren Zeitverzögerung für die Flüssigkeit vom Verlassen des einen Funktionsbereiches bis zum Erreichen des anderen Funktionsbereiches.

Grundsätzlich hat es sich als vorteilhaft erwiesen, wenn die flächige Ausdehnung des Probenaufgabenbereiches größer als die des Nachweisbereiches ist. Im Falle, daß zwischen Probenaufgabe- und Nachweisbereich ein Vorreaktionsbereich angeordnet ist, sollte dieser vorteilhafterweise in seiner flächigen Ausdehnung zwischen der Größe des Probenaufgabe- und des Nachweisbereiches liegen. Grundsätzlich hat es sich als vorteilhaft erwiesen, wenn die Flüssigkeit bei Durchführung des Bestimmungsverfahrens innerhalb der saugfähigen Schicht von einem Funktionsbereich größerer flächiger Ausdehnung zu einem solchen kleinerer flächiger Ausdehnung gelangt.

Die Funktionsbereiche verbindenden Stege können gerade, gebogen, gewinkelt oder in sonstigen möglichen Anordnungen vorliegen. Für den erfindungsgemäßen Testträger hat es sich als besonders vorteilhaft erwiesen, wenn die Stege zwischen den verbundenen Funktionsbereichen gerade sind, daß heißt, die kürzeste Verbindung zwischen zweien in einer bestimmten Entfernung voneinander angeordneten Funktionsbereichen darstellen.

Zur Herstellung eines erfindungsgemäßen Testträgers muß in der saugfähigen Schicht durch Wärmebehandlung das um die Funktionsbereiche und die sie verbindenden Stege herum befindliche Material so verändert werden, daß es nicht mehr saugfähig ist. Wie vorstehend bereits beschrieben, erfolgt dies vorteilhafterweise mit einem Prägewerkzeug, wie beispielsweise einem Stempel oder einer Walze, die in der Prägefläche Vertiefungen aufweisen, die den Funktionsbereichen und den sie verbindenden Stegen entsprechen und umgekehrt die Erhebungen die Flüssigkeit nichtdurchlässigen Begrenzungen um die Funktionsbereiche und die sie verbindenden Stege herum erzeugen. Für den Prägevorgang hat es sich als günstig erwiesen, wenn das saugfähige Material, gegebenenfalls unterlegt mit schmelzbarem Material, das nicht dem saugfähigen Material entspricht, so auf einer ebenen Fläche aufliegt, daß das warme oder mit Wärme erzeugenden Mitteln betriebene Prägewerkzeug das saugfähige Material gegen die ebene Fläche drückt. Wärme und Druck sollten dabei so bemessen sein, daß an den Stellen der Erhebungen des Prägewerkzeugs das saugfähige Material in seiner gesamten Tiefe seine Eigenschaft der Saugfähigkeit verliert. Statt einer ebenen Gegendruckfläche ist es aber auch möglich, eine zweite Prägefläche als Gegendruckfläche einzusetzen, die ein zu dem Prägewerkzeug spiegelbildliches Muster erzeugt. In letzterem Fall wird das saugfähige Material beispielsweise zwischen zwei Prägestempeln bearbeitet, die relativ zueinander so angeordnet sind, daß sie beim Zusammendrücken sowohl von der Unter- als auch von der Oberseite des saugfähigen Materials her paßgenau ein Muster von Funktionsbereichen und sie verbindenen Stegen erzeugen. Bei der Verwendung einer Prägewalze muß die Gegendruckfläche nicht eben, sondern sie kann auch gebogen sein. In jedem Fall muß die Walze beim Abrollen aber in der gesamten Walzenbreite mit den Prägeerhebungen das gewünschte Muster auf der saugfähigen Schicht erzeugen können.

In der Regel wird nach dem Prägevorgang das zur Analytbestimmung notwendige Reagenz an der oder den dafür vorgesehenen Stellen auf das saugfähige Material aufgebracht. Zwar kann dies auch vor dem Prägevorgang geschehen, doch hat sich die Reihenfolge erst prägen dann Reagenz aufbringen als vorteilhaft erwiesen. Das zur Analytbestimmung notwendige Reagenz kann in verschiedenen Verfahren auf die dafür vorgesehenen Funktionsbereiche aufgebracht werden. Als günstig haben sich Druckverfahren, wie beispielsweise Siebdruck, Tampondruck, Ink-Jet, Flexodruck oder Dosierverfahren, wie beispielsweise Nadeldosierung, partieller Walzenauftrag, Roll-Reverse-Walzenauftrag erwiesen. Solche Verfahren sind aus dem Stand der Technik bekannt und brauchen hier nicht näher beschrieben zu werden. Als vorteilhaftes Verfahren hat sich insbesondere das Ink-Jet-Verfahren bewährt.

Das Reagenz zur Analytbestimmung wird entweder vollständig auf den Nachweisbereich aufgebracht, oder die Reagenzbestandteile werden so auf ein oder mehrere Vorreaktionsbereiche und den Nachweisbereich aufgeteilt, daß der oder die ersten Schritte einer mehrstufigen Nachweisreaktion in einem oder mehreren Vorreaktionsbereichen stattfinden und der letzte, das Nachweissignal erzeugende Reaktionsschritt im Nachweisbereich erfolgt. Unter Umständen, kann es aber auch sinnvoll sein, im Nachweisbereich selbst keine chemische Reaktion ablaufen zu lassen, sondern dieses Feld zur Aufkonzentrierung des das Nachweissignal erzeugenden Stoffes zu benutzen. Als besonders vorteilhaft hat es sich für den erfindungsgemäßen Testträger erwiesen, wenn der Nachweis kolorimetrisch erfolgt. Hierbei wird eine Farbe gebildet oder eine Farbänderung tritt auf. Dieser Effekt kann visuell bewertet oder auch quantitativ photometrisch vermessen werden.

Außer dem Auftrag des zur Analytbestimmung notwendigen Reagenzes, kann es sich auch als sinnvoll erweisen, weitere Substanzen auf die Funktionsbereiche aufzubringen. Beispielsweise kann es für die Analytbestimmung aus Vollblut zweckmäßig sein, den Probenaufgabenbereich mit solchen Substanzen zu behandeln, die zu einer Abtrennung der Erythrozyten aus Vollblut führen, so daß nur Plasma oder Serum in die dem Probenaufgabebereich nachgeordneten Funktionsbereiche gelangt. Als besonders vorteilhaft haben sich hierfür Erythrozyten agglutinierende Substanzen erwiesen, wie sie beispielsweise aus EP-B-0 133 895 bekannt sind.

Besitzt die die Funktionsbereiche und die sie verbindenden Stege tragende Schicht nach der Erzeugung der Begrenzung zur Lenkung des Flüssigkeitstransportes eine ausreichende Steifigkeit, kann dieses Material ohne weitere Tragschicht als Testträger zur Analytbestimmung verwendet werden. In der Regel wird es sich jedoch als vorteilhaft erweisen, das mit der Begrenzung zur Lenkung der Probenflüssigkeit versehene, die Funktionsbereiche und die sie verbindenden Stege sowie die zur Bestimmungsreaktion notwendigen Reagenzien tragende Schicht auf einer steifen Tragschicht zu befestigen, damit die Handhabung einfach und problemlos erfolgen kann. Als Tragschicht können durchscheinende oder nicht-durchscheinende steife Materialien, wie Kunststoffolien, Glas, Metall etc. verwendet werden. Besonders vorteilhaft haben sich steife Plastikfolien aus Polystyrol oder Polyester bewährt. Die Befestigung der die Funktionsbereiche tragenden Schicht auf der Tragschicht kann durch Methoden erfolgen, wie sie dem Fachmann für die Herstellung mehrschichtiger Testträger aus dem Stand der Technik bekannt sind. Ein einfaches Verfahren stellt beispielsweise die Befestigung mittels doppelseitig klebendem Band oder mittels Schmelzkleber dar.

Ein besonders einfaches Verfahren zur Erhöhung der Steifigkeit der saugfähigen Schicht wurde bereits vorstehend im Zusammenhang mit der Beschreibung der Erzeugung der den Flüssigkeitsstrom lenkenden Begrenzung im saugfähigen Material beschrieben. Hierbei wird die saugfähige Schicht mit einer in der Wärme verformbaren Schicht an den Stellen, die für Flüssigkeit nicht durchlässig sein sollen so in der Wärme laminiert, daß geschmolzenes Material der in der Wärme verformbaren Schicht die flüssigkeitsundurchlässige Begrenzung in der Saugschicht erzeugt. Durch diese Laminierung, insbesondere eine flächige Verbindung, die lediglich die saugfähigen Bereiche der Funktionsbereiche und der sie verbindenden Stege ausnimmt, wird ein steifer Testträger in einem einfachen Verfahren hergestellt.

Zur Durchführung der Bestimmung eines Analyts in einer Probenflüssigkeit - erfindungsgemäß werden insbesondere Körperflüssigkeiten, wie Blut, Plasma, Serum oder Urin untersucht, es sind jedoch auch alle anderen Arten von Flüssigkeiten möglich - wird Probenflüssigkeit auf das Probenaufgabefeld aufgegeben oder der Testträger mit dem Probenaufgabenfeld in die zu untersuchende Probenflüssigkeit eingetaucht.

Bei der Untersuchung von Blut, Plasma oder Serum wird die Probe üblicherweise auf das Probenaufgabefeld aufgegeben. Bei der Untersuchung von Urin wird der Testträger normalerweise in die zu untersuchende Flüssigkeit eingetaucht.

Nach Aufgabe der zu untersuchenden Probe auf das Probenaufgabefeld oder nach Eintauchen des Testträgers in die zu untersuchende Flüssigkeit breitet sich die Flüssigkeit im Probenaufgabefeld radial aus, bis sie an die thermisch erzeugten Begrenzungen stößt. Dort wo die Flüssigkeit nicht an Begrenzungen stößt, wandert die Flüssigkeit in trockenes saugfähiges Material hinein. Über ein oder mehrere gegebenenfalls vorliegende Vorreaktionsbereiche und die Funktionsbereiche verbindenden Stege gelangt so die Flüssigkeitsfront schließlich in den Nachweisbereich. Ist die saugfähige Schicht im Bereich des Nachweisfeldes vollständig mit Flüssigkeit vollgesaugt und besteht keine weitere Möglichkeit zur Ausbreitung der Flüssigkeit mehr, kommt der Flüssigkeitstransport zum Stillstand. Unter Umständen kann es jedoch auch sinnvoll sein, hinter den Nachweisbereich verbunden mit diesem über einen saugfähigen Steg noch einen leeren Funktionsbereich anzuordnen, so daß Flüssigkeit durch den Nachweisbereich hindurchgesaugt werden kann. Ein solches dem Nachweisbereich nachgeordnetes Feld könnte seiner Funktion entsprechend etwa als Saugfeld bezeichnet werden. Selbstverständlich ist es auch möglich, mehrere Saugfelder dem Nachweisbereich nachzuordnen. Dies könnte beispielsweise so geschehen, daß der Nachweisbereich über einen saugfähigen Steg mit einem ersten Saugfeld verbunden ist und dieses erste Saugfeld wiederrum über einen weiteren saugfähigen Steg mit einem zweiten Saugfeld. Möglich ist aber auch, daß der Nachweisbereich über zwei saugfähige Stege direkt mit zwei Saugfeldern verbunden ist.

So wie für die geometrische Anordnung der Funktionsbereiche zur Bestimmung eines Analyts in einer Probenflüssigkeit viele Möglichkeiten gegeben sind, ist es auch möglich, auf einem erfindungsgemäßen Testträger mehrere Analyte gleichzeitig nachzuweisen. Hierzu können auf einem einzigen Testträger mehrere Probenaufgabebereiche vorhanden sein, die jeder für sich über saugfähige Stege mit Nachweisbereichen gegebenenfalls über Vorreaktionsbereiche mittels saugfähiger Stege miteinander in Verbindung stehen, ohne daß die jeweiligen Funktionsbereiche zur Bestimmung eines Analyts mit den Funktionsbereichen zur Bestimmung eines anderen Analyts miteinander verbunden sind. In einem solchen Fall würden auf einem einzigen Testträger mehrere Probenaufgabebereiche mit den bisher beschriebenen Möglichkeiten zur Anordnung der übrigen Funktionsbereiche unabhängig nebeneinander vorliegen.

Auf einem erfindungsgemäßen Testträger zur Bestimmung mehrerer Analyte in einer Probenflüssigkeit ist es aber auch möglich, daß ein gemeinsamer Probenaufgabenbereich mit den jeweils übrigen Funktionsbereichen die zur Bestimmung mehrerer Analyte nebeneinander notwendig sind, über saugfähige Stege in Verbindung steht. Ausgehend vom gemeinsamen Probenaufgabenbereich findet so praktisch eine Verzweigung zu den für die Bestimmung mehrerer verschiedener Analyte notwendigen Funktionsbereichen statt, die ihrerseits nicht miteinander in Verbindung stehen. Die einzige gemeinsame Stelle ist der Probenaufgabebereich. Unter Umständen ist es auch möglich, daß nicht nur der Probenaufgabebereich sondern auch ein oder mehrere Vorreaktionsbereiche von der auf mehrere Analyte zu untersuchenden Probenflüssigkeit gemeinsam genutzt werden, bevor die Verzweigung in die Funktionsbereiche zur Bestimmung der einzelnen Analyte erfolgt. Beispielsweise könnte es möglich sein, Vollblut auf einen gemeinsamen Probenaufgabebereich aufzugeben, welcher über einen saugfähigen Steg zu einem Vorreaktionsbereich gelangt, welcher mit Erythrozyten agglutinierenden Substanzen versehen ist, so daß ausgehend von diesem Vorreaktionsbereich Plasma oder Serum zur weiteren Reaktion gelangt. Plasma oder Serum könnte ausgehend von diesem Vorreaktionsbereich in Funktionsbereiche zur Bestimmung der einzelnen Analyten verzweigt werden, wobei die nachfolgenden Funktionsbereiche zur Bestimmung der einzelnen Analyten untereinander nicht mehr in Verbindung stehen.

Ein Testträger zur Bestimmung mehrerer Analyte in einer Probenflüssigkeit kann auch so aufgebaut sein, daß mehrere erfindungsgemäße Testträger der vorbeschriebenen Art zur Bestimmung jeweils eines einzigen Analyts schichtenförmig so übereinander angeordnet sind, daß sie über die jeweiligen Probenaufgabefelder miteinander in Verbindung stehen, ansonsten aber voneinander so getrennt sind, daß Flüssigkeit nicht von einer saugfähigen Schicht in eine andere gelangen kann.

Anhand von Abbildungen sollen Ausführungsformen des erfindungsgemäßen Testträgers näher erläutert werden.

Fig. 1a und 1b sowie Fig. 2a und Fig. 3a zeigen einen Querschnitt durch einen Funktionsbereich auf einem erfindungsgemäßen Testträger mit einem Querschnitt durch eine Prägevorrichtung zur Erzeugung dieses Funktionsbereiches.

Fig. 2b und 3b zeigen jeweils einen Querschnitt durch einen Funktionsbereich auf einem erfindungsgemäßen Testträger.

Fig. 4, 5a und 6 zeigen in Aufsicht verschiedene Ausführungsformen für erfindungsgemäße Testträger zur Bestimmung eines Analyts.

Fig. 5b zeigt in schräger, perspektivischer Aufsicht einen erfindungsgemäßen Testträger zur Bestimmung eines Analyts zusammen mit einer zugehörigen Prägevorrichtung.

Fig. 7 - 9 zeigen in Aufsicht Ausführungsformen für erfindungsgemäße Testträger zur Bestimmung mehrerer Analyte in einer Schicht.

Fig. 10 zeigt in schräger, perspektivischer Aufsicht schematisch den Aufbau eines erfindungsgemäßen Testträgers zur Bestimmung von zwei Analyten in zwei Schichten.

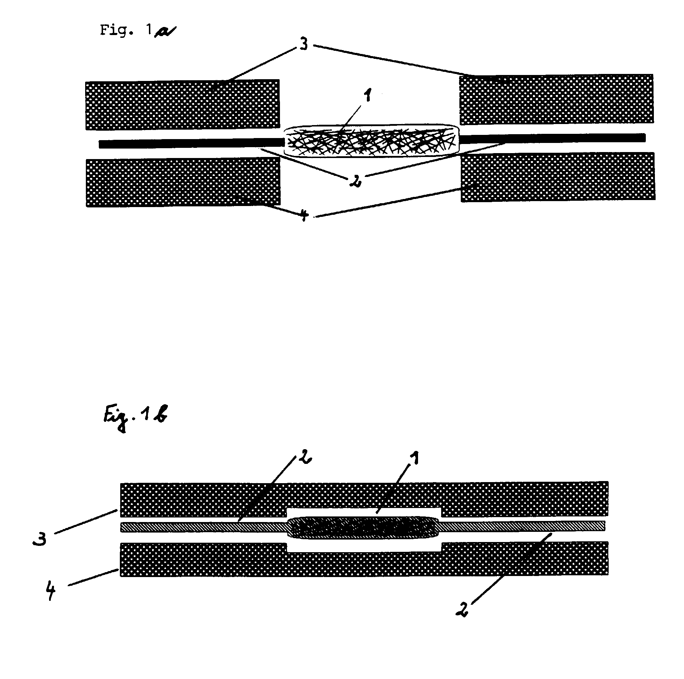

In Fig. 1a und b ist dargestellt, wie aus einem saugfähigen Vlies, das zwischen den warmen Erhebungen (3) und (4) zweier Prägeplatten zweier Stempel zusammengedrückt wird, die für Flüssigkeit nicht durchlässige Begrenzung (2) entstanden ist und der Funktionsbereich (1) als saugfähiger Bereich unverändert geblieben ist.

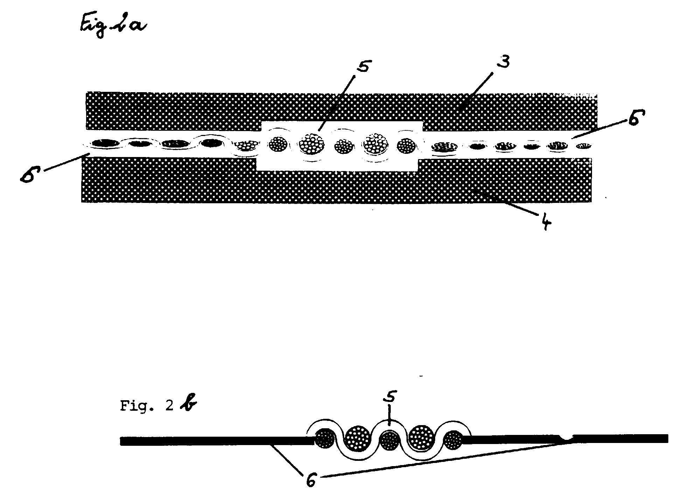

In Fig. 2a und b ist als saugfähige Schicht ein Gewebe dargestellt, aus dem bei Wärmebehandlung unter Druck mit den Prägeplatten (3) und (4) zweier Stempel die für Flüssigkeit undurchlässige Begrenzung (6) um den Funktionsbereich (5) entstanden ist. Innerhalb des Funktionsbereiches (5) sind die regelmäßig angeordneten und miteinander verwebten Fasern erkennbar.

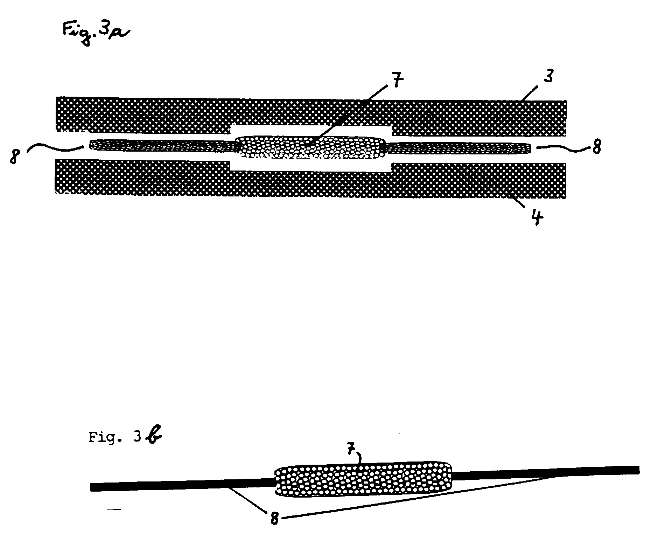

In Fig. 3a und b ist ein Querschnitt durch einen erfindungsgemäßen Testträger dargestellt, bei dem die saugfähige Schicht aus einer Membran besteht, deren Struktur im Bereich des Funktionsbereiches (7) unverändert erhalten geblieben ist, während die Begrenzung (8) unter dem Einfluß von Wärme mit den Prägeplatten (3) und (4) zweier Stempel so zusammengedrückt worden ist, daß dieser Bereich für Flüssigkeit nicht mehr durchlässig ist.

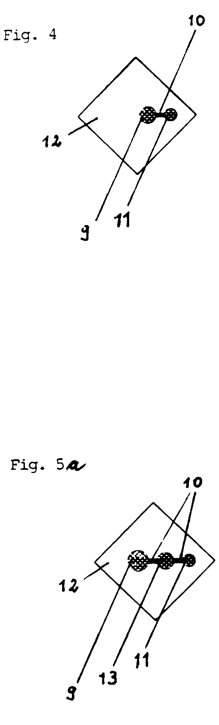

In Fig. 4 ist in Aufsicht ein erfindungsgemäßer quadratischer Testträger dargestellt, bei dem der Probenaufgabebereich (9) mit dem Nachweisbereich (11) über einen saugfähigen Steg (10) miteinander in Verbindung steht. Die Funktionsbereiche (9) und (11) sowie der saugfähige Steg (10) sind insgesamt von Material (12) umgeben, das durch Wärmebehandlung nicht saugfähig gemacht wurde und in das keine Flüssigkeit aus den saugfähigen Bereichen hineingelangt. Der Nachweisbereich (11) ist gegenüber dem Probenaufgabebereich (9) kleiner. Der saugfähige Steg (10) ist gegenüber den Durchmessern der Funktionsbereichen (9) und (11) dünn.

In Fig. 5a ist ein erfindungsgemäßer Testträger dargestellt, der sich von dem in Fig. 4 dargestellten insofern unterscheidet, als zwischen dem Probenaufgabebereich (9) und dem Nachweisbereich (11) ein Vorreaktionsbereich (13) angeordnet ist, der über saugfähige Stege (10) sowohl mit dem Probenaufgabebereich (9) als auch mit dem Nachweisbereich (11) verbunden ist. Die Größe des Vorreaktionsbereiches (13) liegt in seiner flächigen Ausdehnung zwischen derjenigen des Probenaufgabebereiches (9) und der des Nachweisbereiches (11).

In Fig. 5b ist eine längliche Variante des Testträgers gemäß Fig. 5a mit Funktionsbereichen (9,13 und 11) und diese verbindenden Stegen (10) zusammen mit einer entsprechenden Prägeplatte (3) dargestellt. Die Vertiefungen (9', 11' und 13' sowie 10') der Prägeplatte entsprechen den saugfähigen Funktionsbereichen (9, 11 und 13) sowie Stegen (10) des Testträgers.

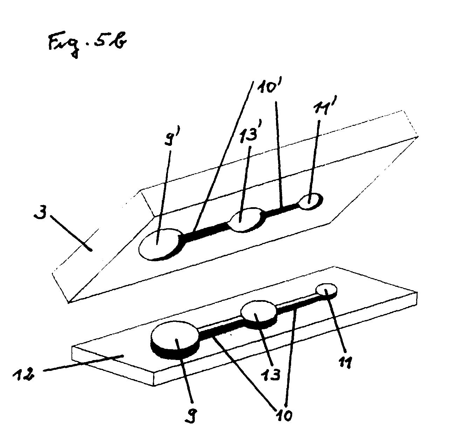

In Fig. 6 ist die Aufsicht auf einen erfindungsgemäßen Testträger dargestellt, der sich von dem in Fig. 5a dargestellten Testträger dadurch unterscheidet, daß der Nachweisbereich (11) mit zwei Saugfeldern (14) über saugfähige Stege (10) verbunden ist. Probe, die auf den Probenaufgabenbereich (9) aufgegeben wird, gelangt über Steg (10) in den Vorreaktionsbereich (13) und von dort über den saugfähigen Steg (10) in den Nachweisbereich (11). Bei ausreichender Flüssigkeitsmenge breitet sich Flüssigkeit vom Nachweisbereich (11) aus über die Stege (10) in die Saugfelder (14) aus.

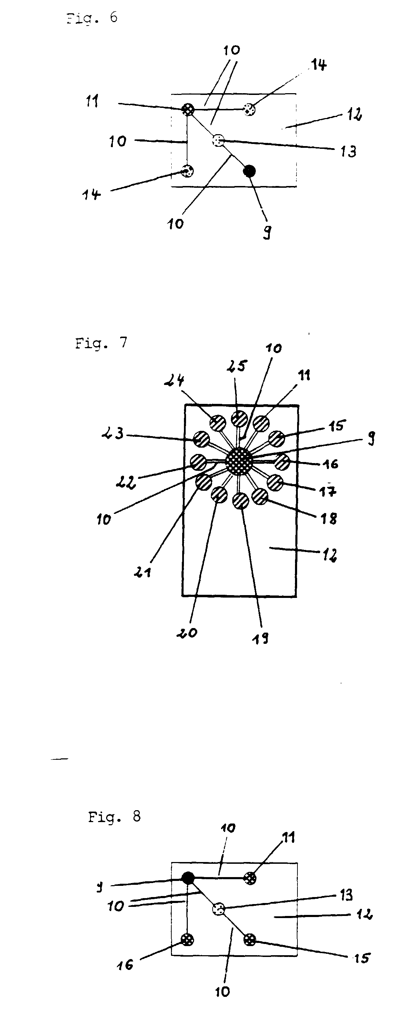

In Fig. 7 ist in Aufsicht ein erfindungsgemäßer Testträger zur Bestimmung mehrerer Analyten in einer Schicht dargestellt. Um einen Probenaufgabenbereich (9) sind über saugfähige Stege (10) Nachweisbereiche für verschiedene Analyte (11, 15, 16, 17, 18, 19, 20, 21, 22, 23, 24, 25) angeordnet. So ist aus einer Probe gleichzeitig die Bestimmung mehrerer Parameter möglich.

Fig. 8 zeigt eine andere Möglichkeit einer geometrischen Anordnung für Funktionsbereiche in einem erfindungsgemäßen Testträger zur Bestimmung mehrerer Analyte in einer Schicht. Der Probenaufgabenbereich (9) ist über saugfähige Stege (10) mit den Nachweisbereichen (11) und (16) für zwei Analyte verbunden. Zur Bestimmung eines dritten Analyts bedarf es einer Vorreaktion. Deshalb ist Probenaufgabenbereich (9) über einen saugfähigen Steg mit dem Vorreaktionsbereich (13) verbunden, welcher seinerseits über einen saugfähigen Steg (10) mit dem Nachweisbereich für den dritten Analyten (15) verbunden ist. So können aus einer Probe drei Parameter simultan bestimmt werden. Eine solche Anordnung könnte sich beispielsweise für die Bestimmung von Triglycerid, Cholesterin und HDL-Cholesterin in Blut, Plasma oder Serum eignen.

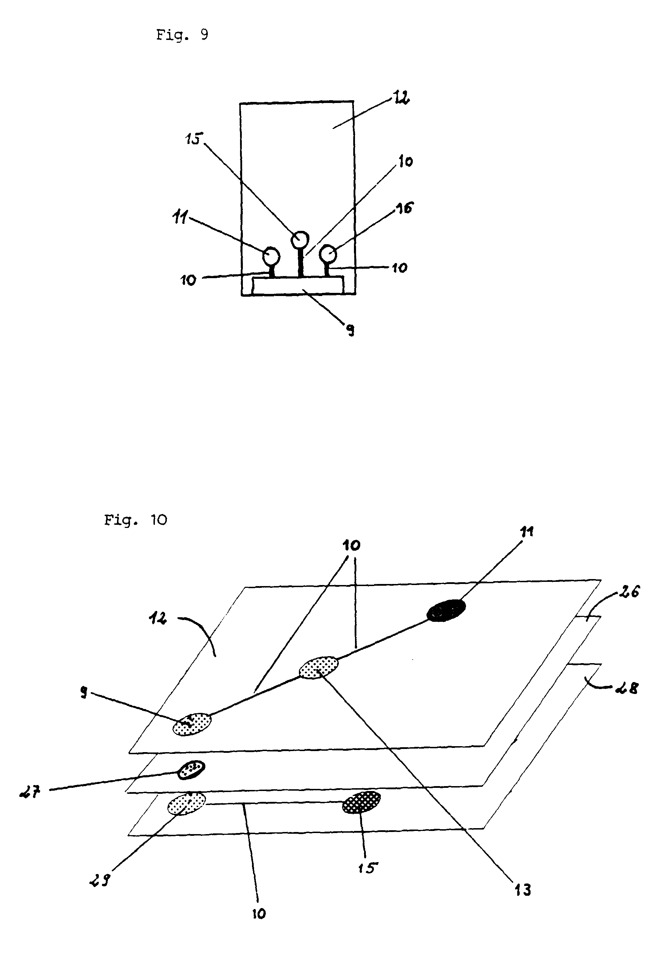

Fig. 9 zeigt einen Eintauchteststreifen zur Bestimmung mehrerer Analyte in einer Probe. Über den Probenaufgabenbereich (9) kann beim Eintauchen des Teststreifens in die zu untersuchende Probenflüssigkeit Flüssigkeit über die Stege (10) in die Nachweisbereiche (11, 15, 16) aufsteigen.

In Fig. 10 ist ein erfindungsgemäßer Testträger zur Bestimmung von zwei Analyten in zwei Schichten dargestellt. In Schicht (12), die nur im Bereich der Funktionsbereiche (9, 13, 11) und der Stege (10) saugfähig ist, ansonsten aber Flüssigkeit nicht transportieren kann, kann flüssige Probe auf den Probenaufgabebereich (9) aufgegeben werden, von wo Probenflüssigkeit über die Stege (10) durch den Vorreaktionsbereich (13) in den Nachweisbereich (11) gelangt, wo der erste Analyt bestimmt wird. Vom Probenaufgabebereich (9) dringt Flüssigkeit gleichzeitig durch die außer in Feld (27) nicht für Flüssigkeit durchlässige Sperrschicht (26) und erreicht in Schicht (28), die nur im Bereich der Funktionsbereiche (29, 15) und des Steges (10) saugfähig ist, ansonsten aber Flüssigkeit nicht transportieren kann, den dortigen Probenaufgabebereich (29). Von dort wird die zu untersuchende Flüssigkeit über den saugfähigen Steg (10) in den Nachweisbereich (15) transportiert. Dort erfolgt der Nachweis des zweiten Analyts. Wenn die Schichten (12, 26 und 28) transparent sind, können kolorimetrische Nachweisreaktionen in den Nachweisbereichen (11) und (15) von einer Seite des Testträgers aus gleichzeitig festgestellt werden. Wenn Schicht (26) nicht transparent ist, kann die Nachweisreaktion des einen Analyts von einer Seite des Testträgers und die Nachweisreaktion des anderen Analyts von der gegenüberliegenden Seite des Testträgers her beobachtet werden.

Wie der vorstehenden Beschreibung zu entnehmen ist, zeichnet sich der erfindungsgemäße Testträger durch einen prinzipiell sehr einfachen Aufbau aus. Entsprechend einfach ist die Herstellung. Gleichzeitig bietet dieser Aufbau eine Vielzahl von Variationsmöglichkeiten für Nachweise verschiedener Analyte, wobei zur unterschiedlichen geometrischen Anordnung der Funktionsbereiche nur unterschiedliche Prägestempel oder -walzen erforderlich sind. Besonders vorteilhaft ist vor allem, daß ein erfindungsgemäßer Testträger nicht nur in Teilschritten, sondern durchaus auch in einer kontinuierlich arbeitenden Produktionslinie erfolgen kann.

Eine Fertigungsanlage für erfindungsgemäße Testträger, die aus folgenden Einrichtungen besteht, hat sich besonders bewährt:
- Prägeeinrichtung mit Prägestempeln oder Prägewalzen
- Reagenzapplikationsstation zur Aufbringung der notwendigen Reagenzien auf die Funktionsbereiche mittels Ink-Jet
- Trockenkanal, vorzugsweise IR- oder Mikrowellentrockner,
- Vereinzelungseinrichtung, die die einzelnen Testträger aus dem bearbeitenden Materialband schneidet.

Angeschlossen kann direkt eine Verpackungseinheit sein.

Da die äußere Form der erfindungsgemäßen Testträger weitgehend unabhängig von der Funktion der Nachweisreaktion gestaltet werden kann, sind Geometrien möglich, die einer automatischen Zuführung und Handhabung in Analysenautomaten entgegenkommen.

Das Probevolumen und demzufolge auch die erforderlichen Reagenzmengen können sehr klein gehalten werden. So sind in der Regel, abhängig von saugfähigem Material und gewählter Geometrie Probevolumina zwischen 3 und 30 µl ausreichend.

Die Erfindung soll durch die folgenden Beispiele noch weiter erläutert werden, ohne daß diese Beispiele die Erfindung auf die konkreten Ausführungsformen beschränken sollen.

### Beispiel 1

### Glucosebestimmung

### a) Herstellung eines Testträgers gemäß Fig. 4

Verwendet wurde ein saugfähiges Faservlies, bestehend aus
- 30: Teile Polyesterfasern
- 20: Teile Zellwollefasern
- 30: Teile Copolyesterfasern (Schmelzkleberfasern Grilene K 170 der Firma Ems-Grilon SA, Domat/Ems, Schweiz)
- 20: Teile Polyvinylalkoholfasern
mit einem Flächengewicht von 140 g/m² und einer Dicke von 0,5 mm.

Das Vlies wurde auf einer als Papiermaschine eingesetzten Schrägsiebmaschine (Fa. Voith, Heidenheim, Deutschland) hergestellt. Hierzu wurden die in Wasser suspendierten Fasern auf ein Schrägsieb gepumpt. Während die Flüssigkeit abfloß bzw. durch Vakuum abgesaugt wurde, orientierten sich die Fasern auf der Sieboberfläche und wurden als Vlies über einem Trockenzylinder getrocknet. Die Trocknung fand bei 125° C statt bis eine Endfeuchte von 0,5 - 1,5 Gew-% erreicht war.

Die für die Prägung eingesetzten Prägestempel der Abmessung 40 x 40 x 20 mm bestanden aus Messing.

In die Stempel war die Prägeform, zwei kreisförmige Aussparungen, verbunden durch einen Steg, eingraviert. Eine kreisförmige Aussparung hatte den Durchmesser 6 mm, die andere kreisförmige Aussparung hatte den Durchmesser 4 mm. Die Aussparung für den Verbindungssteg hatte eine Breite von 2 mm und eine Länge von 4 mm. Die Tiefe der Aussparung im Prägestempel betrug 4 mm.

Für die Prägung des saugfähigen Materials wurde der Prägestempel auf 210° C vorgeheizt; die Prägung selbst wurde mit einem Anpreßdruck von 2 bar (0,2 Mpa) während einer Anpreßzeit von 2 Sekunden durchgeführt.

### b) Imprägnierung

Zur Imprägnierung des Nachweisbereiches (11) mit dem zur Bestimmung von Glucose notwendigen Reagenz werden dort 10 µl einer acetonischen Lösung mit
- 1 Gew.-% 3,3',5,5'-Tetramethylbenzidin (TMB) und
- 1,2 Gew.-% Dioctylnatrium-Sulfosuccinat (DONS)
aufpipettiert und 10 Minuten bei 60° C getrocknet.

Danach werden 10 µl einer Lösung von
- Glucose-Oxidase (250 kU/l) und
- Peroxidase (500 kU/l)
in Phosphatpufferlösung (pH 7,0; 0,1 mol/l) ebenralls auf den Nachweisbereich (11) appliziert und 30 Minuten bei 60° C getrocknet.

### c) Durchführung der Glucosebestimmung

Nach Aufgabe von ca. 30 µl glucosehaltiger Kontrollseren auf den Probenaufgabebereich (9) entsteht im Nachweisbereich (11) innerhalb von 10 Sekunden eine konzentrationssabhängig unterschiedlich intensive homogene blaue Farbe.

### Beispiel 2:

### Cholesterinbestimmung

Auf den Nachweisbereich (11) eines wie in Beispiel 1 a) hergestellten Testträgers gemäß Fig. 4 werden zur Imprägnierung mit dem zur Bestimmung von Cholesterin notwendigen Reagenz 10 µl einer acetonischen Lösung mit
- 1 Gew.% TMB und
- 1,2 Gew.% DONS
aufpipettiert und 10 Minuten bei 60° C getrocknet.

Auf den getrockneten, mit Indikator versehenen Nachweisbereich (11), werden 10 µl folgender Enzymlösung aufpippetiert:
- Cholesterin-Esterase (500 kU/l)
- Cholesterin-Oxidase (50 kU/l)
- Peroxidase (500 kU/l)
- MgCl₂ . 6H₂O (25 mmol/l)
in Phosphatpuffer (pH 7,0; 0,1 mol/l).

Anschließend wird 30 Minuten bei 60° C getrocknet.

Bei Applikation einer Konzentrationsreihe cholesterinhaltiger Kontrollseren auf den Probenaufgabebereich entsteht nach ca. 10 Sekunden im Nachweisbereich (11) eine konzentrationsbedingt unterschiedlich intensive homogene blaue Reaktionsfarbe.

## Patentansprüche

1. Testträger zur Bestimmung eines Analyts in einer flüssigen Probe enthaltend eine Schicht eines flächig ausgebildeten Materials mit einem saugfähigen Probenaufgabebereich und einem saugfähigen Nachweisbereich, der für die Bestimmung des Analyten erforderliches Reagenz enthält, sowie gegebenenfals weitere saugfähige Funktionsbereiche, wobei sich die räumlich getrennten saugfähigen Bereiche innerhalb von Begrenzungen, durch die hindurch kein Flüssigkeitstransport stattfinden kann, auf dem flächig ausgebildeten Material befinden,
dadurch gekennzeichnet, daß
sich Probenaufgabebereich und Reagenz enthaltender Nachweisbereich sowie gegebenenfalls weitere saugfähige Funktionsbereiche auf der gleichen Schicht des flächig ausgebildeten Materials befinden, sowie
Probenaufgabe- und Reagenz enthaltender Nachweisbereich sowie gegebenenfalls weitere saugfähige Funktionsbereiche durch saugfähige Stege voneinander getrennt sind,
wobei Probenaufgabe-, Reagenz enthaltender Nachweisbereich sowie gegebenenfalls weitere saugfähige Funktionsbereiche und saugfähige Stege durch eine Begrenzung gebildet und vom Rest des flächig ausgebildeten Materials so abgetrennt sind, daß zwischen Probenaufgabe- und Reagenz enthaltendem Nachweisbereich über den oder die saugfähigen Stege sowie gegebenenfalls weitere saugfähige Funktionsbereiche ein Flüssigkeitstransport erfolgen kann, Flüssigkeitstransport aus einem der Bereiche oder den Stegen in das außerhalb der Begrenzung liegende restliche flächig ausgebildete Material jedoch verhindert ist.

2. Testträger gemäß Anspruch 1, dadurch gekennzeichnet, daß die Begrenzungen durch die hindurch kein Flüssigkeitstransport stattfinden kann, innerhalb des flächig ausgebildeten Materials durch Wärmebehandlung des saugfähigen, flächig ausgebildeten Materials selbst gebildet worden sind.

3. Testträger gemäß Anspruch 1, dadurch gekennzeichnet, daß die Begrenzungen aus einem von dem flächig ausgebildeten, saugfähigen Schichtenmaterial verschiedenen Material bestehen.

4. Testträger gemäß Anspruch 1, dadurch gekennzeichnet, daß der Probenaufgabebereich größer als der Nachweisbereich ist.

5. Testträger gemäß einem der Ansprüche 1 - 4, dadurch gekennzeichnet, daß das flächig ausgebildete Material in den saugfähigen Bereichen faserig ist.

6. Testträger gemäß einem der Ansprüche 1 - 4, dadurch gekennzeichnet, daß das flächig ausgebildete Material in den saugfähigen Bereichen porös ist.

7. Verfahren zur Herstellung eines Testträgers gemäß einem der Ansprüche 1 - 6, dadurch gekennzeichnet, daß in einer Schicht eines saugfähigen Materials durch Wärmebehandlung das um die saugfähigen Bereiche und die sie verbindenden Stege herum befindliche Material so verändert wird, daß es nicht mehr saugfähig ist.

8. Verfahren gemäß Anspruch 7, dadurch gekennzeichnet, daß die Wärmebehandlung mit Ultraschall durchgeführt wird.

9. Verfahren gemäß einem der Ansprüche 7 und 8, dadurch gekennzeichnet, daß das saugfähige, flächig ausgebildete Material selbst in der Wärme verformbar ist.

10. Verfahren gemäß einem der Ansprüche 7 und 8, dadurch gekennzeichnet, daß durch lokale Wärmebehandlung eines in der Wärme verformbaren Materials das unter dem saugfähigen, flächig ausgebildeten Material liegt, letzteres lokal so verändert wird, daß es dort nicht mehr saugfähig ist.

## Claims

1. Analytical device for determining an analyte in a liquid sample comprising
a layer of a flat shaped material with an absorbent sample application area and an absorbent detection area containing reagent necessary to determine the analyte and also, if desired,
further absorbent functional areas wherein the spatially separated absorbent areas are located on the flat shaped material within a boundary through which transport of sample liquid cannot take place,
characterised in that
sample application area and detection area containing reagent as well as further optional absorbent functional layers are located on the same layer of the flat shaped material and
sample application area and detection area containing reagent and further optional absorbent functional areas are separated from one another by absorbent connectors,
wherein a boundary forms the sample application area, detection area containing reagent as well as further optional absorbent functional areas and absorbent connectors and separates them from the remainder of the flat shaped material in such a way that liquid transport can take place between sample application area and detection area containing reagent via the absorbent connector or connectors and, if desired, further absorbent functional areas but prevents liquid transport from one of the areas or the connectors into the remaining flat shaped material located outside the boundary.

2. Analytical device as claimed in claim 1, wherein the boundary through which no liquid transport can take place has been formed within the flat shaped material by heat treatment of the absorbent flat shaped material itself.

3. Analytical device as claimed in claim 1, wherein the boundary consists of a material which is different from the flat shaped absorbent layer material.

4. Analytical device as claimed in claim 1, wherein the sample application area is larger than the detection area.

5. Analytical device as claimed in one of the claims 1-4, wherein the flat shaped material is fibrous in the absorbent areas.

6. Analytical device as claimed in one of the claims 1-4, wherein the flat shaped material is porous in the absorbent areas.

7. Process for the production of an analytical device as claimed in one of the claims 1 - 6, wherein in a layer of an absorbent material, the material which is located around the absorbent areas and around the connectors which link them is changed in such a way that it is no longer absorbent.

8. Process as claimed in claim 7, wherein the heat treatment is carried out with ultrasound.

9. Process as claimed in one of the claims 7 and 8, wherein the absorbent flat shaped material can itself be deformed by heat.

10. Process as claimed in one of the claims 7 and 8, wherein by local heat treatment of a heat-deformable material which is located under the absorbent flat shaped material the latter is changed locally in such a way that it is no longer absorbent.

## Revendications

1. Support de test pour déterminer un analyte dans un prélèvement liquide, contenant une couche d'un matériau formé en surface avec une zone absorbante de réception de prélèvements et une zone absorbante de détection, qui contient un réactif nécessaire à la détermination de l'analyte, ainsi que, le cas échéant, des zones absorbantes fonctionnelles supplémentaires, dans lequel les zones absorbantes spatialement séparées se trouvent à l'intérieur de délimitations, à travers lesquelles aucun transport de liquide ne peut avoir lieu, sur le matériau formé en surface,
caractérisé en ce que, la zone de réception des prélèvements et la zone de détection contenant le réactif ainsi que, le cas échéant, les zones absorbantes fonctionnelles supplémentaires, se trouvent sur la même couche du matériau formé en surface, et que la zone de réception des prélèvements et la zone de détection contenant le réactif ainsi que, le cas échéant, les zones fonctionnelles supplémentaires absorbantes, sont séparées les unes des autres par des passerelles absorbantes,
dans lequel la zone de réception des prélèvements et la zone de détection contenant le réactif ainsi que, le cas échéant, les zones fonctionnelles supplémentaires absorbantes, et les passerelles absorbantes sont configurées par une délimitation et sont séparées du reste du matériau formé en surface de sorte qu'un transport de liquide puisse avoir lieu par la ou les passerelles absorbantes ainsi que, le cas échéant, par les zones absorbantes fonctionnelles supplémentaires entre la zone de réception de prélèvements et la zone de détection contenant le réactif, en empêchant toutefois un transport de liquide à partir d'une des zones ou des passerelles vers le matériau formé en surface restant se trouvant en dehors de la délimitation.

2. Support de test selon la revendication 1, caractérisé en ce que les délimitations, à travers lesquelles il ne peut avoir lieu aucun transport de liquide, se sont formées toutes seules à l'intérieur du matériau formé en surface au moyen d'un traitement thermique du matériau formé en surface.

3. Support de test selon la revendication 1, caractérisé en ce que les délimitations sont constituées d'une matière différente de la matière en couches absorbante formée en surface.

4. Support de test selon la revendication 1, caractérisé en ce que la zone de réception des prélèvements est plus grande que la zone de détection.

5. Support de test selon l'une quelconque des revendications 1 à 4, caractérisé en ce que le matériau formé en surface dans les zones absorbantes est fibreux.

6. Support de test selon l'une quelconque des revendications 1 à 4, caractérisé en ce que le matériau formé en surface dans les zones absorbantes est poreux.

7. Procédé de fabrication d'un support de test selon l'une quelconque des revendications 1 à 6, caractérisé en ce que, dans une couche du matériau absorbant, le matériau se trouvant autour des zones absorbantes et des passerelles qui les relient, est modifié au moyen d'un traitement thermique de façon qu'il ne soit plus absorbant.

8. Procédé selon la revendication 7, caractérisé en ce que le traitement thermique s'effectue à l'aide d'ultrasons.

9. Procédé selon l'une quelconque des revendications 7 et 8, caractérisé en ce que, le matériau absorbant formé en surface est lui-même thermodéformable.

10. Procédé selon l'une quelconque des revendications 7 et 8, caractérisé en ce que, au moyen d'un traitement thermique locale d'un matériau thermodéformable qui se trouve au-dessous du matériau formé en surface, celui-ci est modifié localement de façon qu'il n'y soit plus absorbant.
